# EUROPEAN PATENT APPLICATION

(11) **EP 4 520 256 A1**
(43) Date of publication of application: **12.03.2025**
(21) Application number: 23898210.2
(22) Date of filing: 24.11.2023
(51) Int. Cl.: A61B 5/00, A61B 5/16, G16H 10/20, G16H 50/20, G16H 50/30

(54) **GAZE TRACKING-BASED SOCIAL CONTEXT AWARENESS ABILITY ANALYSIS DEVICE, METHOD, AND COMPUTER PROGRAM**

(30) Priority: 29.11.2022 KR 20220163319
(71) Applicant: Neudive Inc., Daegu 41061 (KR)
(72) Inventor: JU, Ran, Incheon 21056 (KR); CHO, Sung Ja, Seoul 08727 (KR)
(74) Representative: Krauns, Christian
(86) International application number: PCT/KR2023/019055
(87) International publication number: WO 2024/117679

(57) **Abstract**

A cognitive ability analysis device according to an embodiment may include a content unit that provides content in which an area that serves as a clue for determining social situations is pre-designated; a gaze acquisition unit that acquires gaze information of a point where a user gazes at the content; a query unit that presents a question related to the content and acquires an answer from the user; a determination unit that determines user's cognitive ability for social situations based on the gaze information and answer from the user.

## Description

### TECHNICAL FIELD

The present invention relates to a device, method, and computer program for analyzing cognitive ability for social situations based on eye tracking.

### BACKGROUND ART

Autism refers to a disorder in which a person does not form relationships with others and does not develop emotional bonds. The International Classification of Diseases (ICD-10) diagnosed autism as a pervasive developmental disorder throughout development, and categorized pervasive developmental disorders into nine subtypes. Recently, a follow-up study related to this concluded that it is not clinically significant to categorize them separately, and autism is also referred to as the autism spectrum.

Meanwhile, in determining whether a child has autism, a model has been used to check whether the child shows an appropriate level of emotional response to a given specific situation, and to diagnose autism if an appropriate level of response is not shown.

However, because the factors that need to be determined in order to recognize various social situations may vary not only in emotional responses but also in the patterns of social situations, an accurate assessment tool is needed to implement a social skills training program that allows children with autism to accurately recognize and cope with social situations.

### DESCRIPTION OF EMBODIMENTS

### TECHNICAL PROBLEM

Provided is a technology capable of analyzing various information, including the gaze of children with autism, in relation to a social situation context to determine an accurate cognitive state for social situations.

### SOLUTION TO PROBLEM

A cognitive ability analysis device according to an embodiment may include a content unit that provides content in which an area that serves as a clue for determining social situations is pre-designated; a gaze acquisition unit that acquires gaze information of a point where a user gazes at the content; a query unit that presents a question related to the content and acquires an answer from the user; a determination unit that determines user's cognitive ability for social situations based on the gaze information and answer from the user.

In addition, in the content in which an area that serves as a clue is pre-designated, the area may be pre-designated based on coordinate information for an area including some of texts output by the content, or coordinate information for an area including some of images output by the content.

In addition, the content may include images, texts, and voices representing social situations, and the content unit may simultaneously output a voice matching text shown in the content.

In addition, the gaze acquisition unit may acquire coordinate information of a point where the user gazes at the content, time information of the point of gaze, and information about an order in which the point of gaze has moved in time series.

In addition, the gaze acquisition unit may separately specify and store a point where the user gazes at a certain point for a preset time or longer.

In addition, the determination unit may determine whether the area that serves as the clue includes the point where the user gazes at a certain point for a preset time or longer.

Furthermore, the gaze acquisition unit may collect coordinate information of a point where the user gazes at every 10 ms, and may specify a point where a gaze time is 60 ms or longer.

In addition, the determination unit may determine cognitive ability for social situations of the content from user's gaze information based on a preset score criterion for each of gaze coordinate information, gaze time, whether gaze occurred for a preset time or longer, and whether gaze occurred in an area that serves as a clue.

In addition, the determination unit may use a neural network model trained to determine cognitive ability for social situations of content based on each data such as gaze coordinate information, gaze time, whether gaze occurred for a preset time or longer, and whether gaze occurred in an area that serves as a clue.

In addition, when a user's answer to a question related to content is correct, but a cognitive ability score for social situations determined from user's gaze information is below a preset value, the determination unit 140 may determine that the user's answer is incorrect and search for new content to reconfirm user's cognitive ability and provide the new content to the user.

A cognitive ability analysis method performed by a cognitive ability analysis device according to an embodiment may include providing content in which an area that serves as a clue for determining social situations is pre-designated; acquiring gaze information of a point where a user gazes at the content; presenting a question related to the content and acquires an answer from the user; and determining user's cognitive ability for social situations based on the gaze information and answer from the user.

### ADVANTAGEOUS EFFECTS OF DISCLOSURE

According to an embodiment of the present invention, a sophisticated technique may be provided that analyzes the degree and timing of user attention to a clue information area required for social situation recognition, and changes in facial expressions at the time of attention, over time. For example, in cases where group bullying occurs or when several people create a deceptive situation to tease one person, it is impossible to accurately recognize the overall situation by looking at only one person's facial expression, so in a situation created by content, the present invention may determine user's cognitive ability for social situations based on whether a user shows any reaction to clue information areas that may identify the deceptiveness of the situation. As a result, the present invention may serve as an evaluation tool for implementing a social skills training program that enables a user to accurately recognize and cope with various social situations.

### BRIEF DESCRIPTION OF DRAWINGS

FIG. 1 is a functional block diagram of a cognitive ability analysis device for social situations based on eye tracking according to an embodiment of the present invention.
FIG. 2 is an exemplary view of content according to an embodiment of the present invention.
FIG. 3 is an exemplary view of gaze information collected from content according to an embodiment of the present invention.
FIG. 4 is an exemplary view of an operation of providing a question corresponding to content according to an embodiment of the present invention to acquire an answer.
FIG. 5 is an exemplary view of an operation for determining user's cognitive ability for social situations based on user's gaze information and answer according to an embodiment of the present invention.
FIG. 6 is a flowchart of a cognitive ability analysis method performed by the cognitive ability analysis device according to an embodiment of the present invention.

### MODE OF DISCLOSURE

Hereinafter, embodiments of the present invention will be described with reference to the drawings. In the following description, descriptions of a well-known technical configuration in relation to a lead implantation system for a deep brain stimulator will be omitted. For example, descriptions of the configuration/structure/method of a device or system commonly used in deep brain stimulation, such as the structure of an implantable pulse generator, a connection structure/method of the implantable pulse generator and a lead, and a process for transmitting and receiving electrical signals measured through the lead with an external device, will be omitted. Even if these descriptions are omitted, one of ordinary skill in the art will be able to easily understand the characteristic configuration of the present invention through the following description.

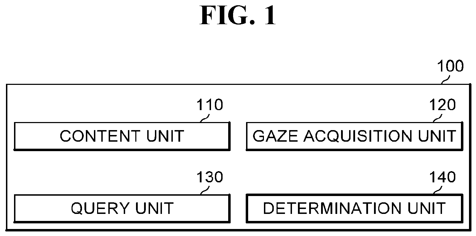

FIG. 1 is a functional block diagram of a cognitive ability analysis device 100 for social situations based on eye tracking (Hereinafter, 'cognitive ability analysis device 100') according to an embodiment of the present invention.

Referring to FIG. 1, the cognitive ability analysis device 100 according to an embodiment of the present invention may include a content unit 110, a gaze acquisition unit 120, a query unit 130, and a determination unit 140. The cognitive ability analysis device 100 according to an embodiment of the present invention may perform overall operations by one or more processors, and the one or more processors may control functional blocks included in FIG. 1 to perform operations described below.

The content unit 110 may provide a user with content for assessing cognitive ability for social situations. Content according to an embodiment may include images, texts, and voices representing social situations, and may simultaneously output a voice matching text shown in the content. For example, the content unit 110 may output content to a user through a display connected to the cognitive ability analysis device 100 by wire or wirelessly. For example, the content unit 110 may provide content to a user terminal connected to the cognitive ability analysis device 100 by wire or wirelessly. The content unit 110 may include a hardware memory for storing content. The content unit 110 may include a communication module for acquiring content from an external device.

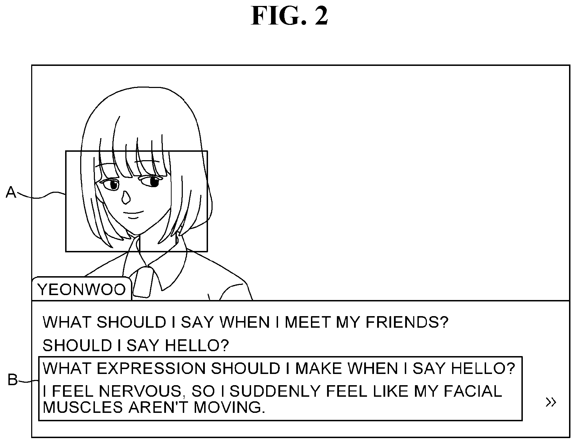

FIG. 2 is an exemplary view of content according to an embodiment of the present invention.

Referring to FIG. 2, according to an embodiment, content may have areas (A and B in FIG. 2) pre-designated as clues for determining social situations. For example, in order to specify the areas, content may have coordinate information pre-specified within an area (A in FIG. 2) that includes some images (e.g., areas of images expressing facial expressions) from among areas where images of the content are output, or coordinate information pre-specified within an area (B in FIG. 2) that includes some texts (e.g., areas of text expressing emotions) from among areas where text of the content is output.

The gaze acquisition unit 120 may acquire gaze information of a point where a user gazes at content.

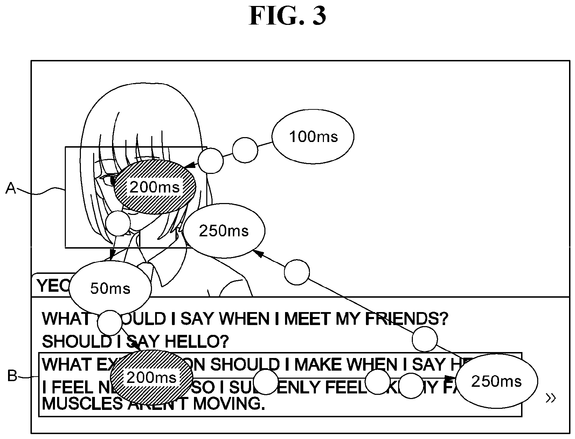

FIG. 3 is an exemplary view of gaze information collected from content according to an embodiment of the present invention.

Referring to FIG. 3, the gaze acquisition unit 120 may acquire coordinate information of a point where a user gazes at the content, time information of the point of gaze, and information about an order in which the point of gaze has moved in time series. For example, the gaze acquisition unit 120 may include a camera module capable of detecting a user's gaze, and may measure coordinate information of a point where a user gazes in units of 10 ms and a gaze retention time of the user at a certain point. The gaze acquisition unit 120 may separately specify and store a point where a user gazes at a certain point for a preset time (e.g., 60 ms) or longer, and at this time, the determination unit 140 may determine whether areas that serve as clues (e.g., A and B in FIG. 2) include the point where a user gazes at a certain point for a preset time (e.g., 60 ms) or longer. The gaze acquisition unit 120 may acquire information about an order in which a user's gaze has moved in time series by storing acquired time information in time series.

The query unit 130 may acquire a user's answer by presenting a question related to the content.

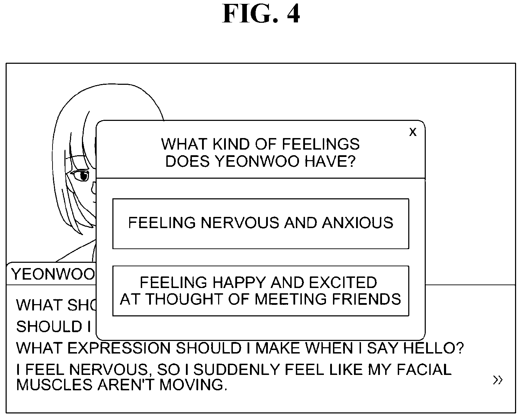

FIG. 4 is an exemplary view of an operation of providing a question corresponding to content according to an embodiment of the present invention to acquire an answer.

Referring to FIG. 4, the query unit 130 may output a question related to social situations included in content and receive an answer to the question from a user.

The determination unit 140 may determine user's cognitive ability for social situations based on user's gaze information and answer.

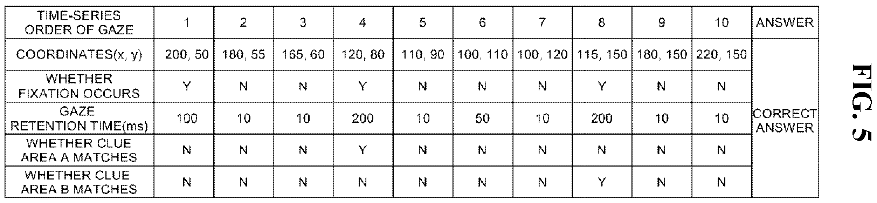

FIG. 5 is an exemplary view of an operation for determining user's cognitive ability for social situations based on user's gaze information and answer according to an embodiment of the present invention.

Referring to FIG. 5, the determination unit 140 may determine cognitive ability for social situations of content from the user's gaze information based on a preset score criterion for each of gaze coordinate information, whether gaze occurred for a preset time (e.g., 60 ms) or longer, gaze time, and whether gaze occurred in areas that serve as clues (e.g., A and B in FIG. 2) according to a time-series order of the user's gaze. In addition, the determination unit 140 may determine the user's cognitive ability for social situations from the user's gaze information by using a neural network model trained to determine cognitive ability for social situations based on each data such as gaze coordinate information, gaze time, whether gaze occurred for a preset time or longer, and whether gaze occurred in an area that serves as a clue.

For example, when a user's answer to a question related to content is correct, but a user's cognitive ability score determined from user's gaze information is below a preset value, the determination unit 140 may determine that the user's answer is incorrect and search for new content to reconfirm user's cognitive ability to re-measure the user's cognitive ability.

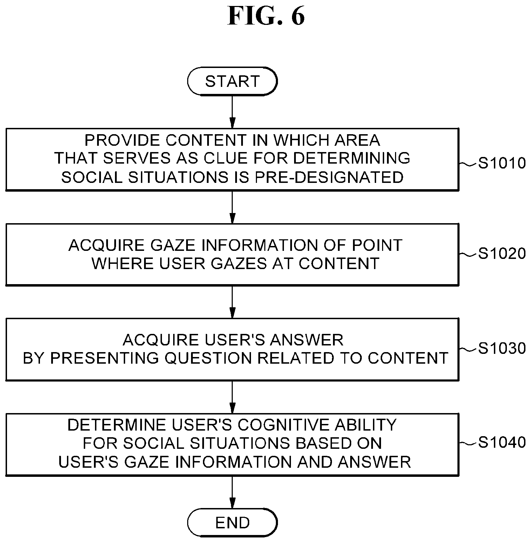

FIG. 6 is a flowchart of a cognitive ability analysis method performed by the cognitive ability analysis device 100 according to an embodiment of the present invention. Each operation of the cognitive ability analysis method according to FIG. 6 may be performed by the cognitive ability analysis device 100 described in FIG. 1, and each operation is described as follows.

In operation S1010, the content unit 110 may provide content in which an area that serves as a clue for determining social situations is pre-designated.

In operation S1020, the gaze acquisition unit 120 may acquire gaze information of a point where a user gazes at the content.

In operation S1030, the query unit 130 may acquire a user's answer by presenting a question related to the content.

In operation S1040, the determination unit 140 may determine user's cognitive ability for social situations based on the user's gaze information and answer.

Meanwhile, in addition to the operations shown in FIG. 6, because the content unit 110, the gaze acquisition unit 120, the query unit 130, and the determination unit 140 described above configure various embodiments to perform the operations described in FIGS. 1 to 5, even in the operations of FIG. 6, a new operation performed by each functional block may be added. Because a configuration of an additional operation and operations of components responsible for each operation to perform respective operations have been described in FIGS. 1 to 5, redundant descriptions will be omitted.

According to the embodiment described above, a sophisticated technique may be provided that analyzes the degree and timing of user attention to a clue information area required for social situation recognition, and changes in facial expressions at the time of attention, over time. For example, in cases where group bullying occurs or when several people create a deceptive situation to tease one person, it is impossible to accurately recognize the overall situation by looking at only one person's facial expression, so in a situation created by content, the present invention may determine user's cognitive ability for social situations based on whether a user shows any reaction to clue information areas that may identify the deceptiveness of the situation. As a result, the present invention may serve as an evaluation tool for implementing a social skills training program that enables a user to accurately recognize and cope with various social situations.

In concluding the detailed description, those skilled in the art will appreciate that many variations and modifications may be made to the preferred embodiments without substantially departing from the principles of the present invention. Therefore, the disclosed preferred embodiments of the invention are used in a generic and descriptive sense only and not for purposes of limitation.

## Claims

1. A cognitive ability analysis device comprising:
a content unit that provides content in which an area that serves as a clue for determining social situations is pre-designated;
a gaze acquisition unit that acquires gaze information of a point where a user gazes at the content;
a query unit that presents a question related to the content and acquires an answer from the user; and
a determination unit that determines user's cognitive ability for social situations based on the gaze information and answer from the user.

2. The cognitive ability analysis device of claim 1, wherein, in the content in which an area that serves as a clue, the area is pre-designated based on coordinate information for an area including some of texts output by the content, or coordinate information for an area including some of images output by the content.

3. The cognitive ability analysis device of claim 2, wherein the content comprises images, texts, and voices representing social situations, and
the content unit simultaneously outputs a voice matching text shown in the content.

4. The cognitive ability analysis device of claim 1, wherein the gaze acquisition unit acquires coordinate information of a point where the user gazes at the content, time information of the point of gaze, and information about an order in which the point of gaze has moved in time series.

5. The cognitive ability analysis device of claim 4, wherein the gaze acquisition unit separately specifies and stores a point where the user gazes at a certain point for a preset time or longer.

6. The cognitive ability analysis device of claim 5, wherein the determination unit determines whether the area that serves as the clue comprises the point where the user gazes at the certain point for the preset time or longer.

7. The cognitive ability analysis device of claim 5, wherein the gaze acquisition unit collects coordinate information of a point where the user gazes at every 10 ms, and specifies a point where a gaze time is 60 ms or longer.

8. The cognitive ability analysis device of claim 1, wherein the determination unit determines cognitive ability for social situations of the content from user's gaze information based on a preset score criterion for each of gaze coordinate information, gaze time, whether gaze occurred for a preset time or longer, and whether gaze occurred in an area that serves as a clue.

9. The cognitive ability analysis device of claim 8, wherein the determination unit uses a neural network model trained to determine cognitive ability for social situations of content based on each data such as gaze coordinate information, gaze time, whether gaze occurred for a preset time or longer, and whether gaze occurred in an area that serves as a clue.

10. The cognitive ability analysis device of claim 8, wherein, when a user's answer to a question related to content is correct, but a cognitive ability score for social situations determined from user's gaze information is below a preset value, the determination unit determines that the user's answer is incorrect and search for new content to reconfirm user's cognitive ability and provide the new content to the user.

11. A cognitive ability analysis method performed by a cognitive ability analysis device, the cognitive ability analysis method comprising:
providing content in which an area that serves as a clue for determining social situations is pre-designated;
acquiring gaze information of a point where a user gazes at the content;
presenting a question related to the content and acquiring an answer from the user; and
determining user's cognitive ability for social situations based on the gaze information and answer from the user.

12. A program stored on a computer-readable recording medium for executing each operation according to the method of claim 11.
